# EUROPEAN PATENT SPECIFICATION

(11) **EP 2 956 766 B1**
(45) Date of publication and mention of the grant of the patent: **02.08.2017**
(21) Application number: 14714352.3
(22) Date of filing: 18.02.2014
(51) Int. Cl.: G01N 27/49, G01N 33/18

(54) **PROBE-HOLDER FOR AMPEROMETRIC SENSOR**
SONDENHALTER FÜR EINEN AMPEROMETRISCHEN SENSOR
SUPPORT DE TUBE À ÉCHANTILLONS POUR UN CAPTEUR AMPÉROMÉTRIQUE

(30) Priority: 18.02.2013 IT RM20130093
(43) Date of publication of application: 23.12.2015
(73) Proprietor: SEKO S.p.A., 02010 Santa Rufina, Cittaducale (RI) (IT)
(72) Inventor: LIVOTI, Stefano, I-02010 Cittaducale (RI) (IT); DAMIANI, Andrea, I-02010 Cittaducale (ri) (IT); CORNACCHIOLA, Sergio, I-02010 Cittaducale (ri) (IT)
(74) Representative: Scilletta, Andrea
(86) International application number: PCT/IB2014/059062
(87) International publication number: WO 2014/125456

(56) References cited:
- WO-A1-02/101400
- WO-A1-2012/085641
- US-A- 4 129 479
- US-B2- 6 896 793

## Description

The present invention relates to a probe-holder configured to house an amperometric sensor for sensing the concentration of at least one specific substance possibly dissolved (e.g., chlorine) in a fluid, optionally water, that flows in the probe-holder, possibly besides other sensing probes for sensing physico-chemical parameters of the fluid, that allows to carry out precise, efficient and reliable measurements of the concentration of said at least one substance.

In the following of the present description, reference will be mainly made to a probe-holding module for amperometric sensor that constitutes an embodiment of the probe-holder for amperometric sensor according to the invention, wherein the probe-holding module for amperometric sensor is assembled in a modular probe-holder applied to water treatment.

However, it must understood that the probe-holder for amperometric sensor according to the invention may be a different probe-holder, for instance a monolithic (i.e. non-modular) probe-holder configured to house only an amperometric sensor or configured to house, besides the amperometric sensor, other sensing probes for sensing physico-chemical parameters of the fluid, such as flow meters, and that the probe-holder for amperometric sensor according to the invention may be applied in any other technological context in which the physico-chemical parameters of any fluid, even different from water, must be monitored and/or controlled, still remaining within the scope of protection of the present invention as defined in the attached claims.

It is known that in water treatment it is essential to sense physico-chemical parameters of the same water, in particular the concentration of possibly dissolved specific substances (e.g., chlorine), besides other parameters such as, for instance, the volumetric flow rate or the pH value. To this end, sensing probes are used, which are arranged along a sensing path in which water that is to undergo sensing at least partially flows. In particular, such sensing probes are housed in a dedicated compartment of a monolithic probe-holder, usually of plastic material, provided with an inlet and an outlet, that is part of such sensing path.
the inventors have developed a modular probe-holder composed of two or more probe-holding modules, with reference to which the invention will be illustrated in the following.

For sensing the concentration of specific substances possibly dissolved in the fluid, amperometric sensors are commonly used, as illustrated for instance by the prior art documents US4129479, WO 02/101400 A1 and WO 2012/085641 A1.

However, the solutions proposed in the prior art suffer from some drawbacks mainly due to a non-efficient agitation of the solute in the fluid in correspondence of the amperometric sensor, that causes the measurement to be not completely precise, efficient and reliable.

It is an object of this invention, therefore, to allow to carry out precise, efficient and reliable measurements of the concentration of at least one substance possibly dissolved in a fluid, optionally water.

It is specific subject-matter of the present invention a probe-holder, having a first and a second side walls, configured to house an amperometric sensor in a side hollow seat, the probe-holder comprising an inlet and an outlet and a longitudinal duct connected downstream of the side hollow seat, the probe-holder being configured to receive a flow of a fluid from the inlet to the outlet, the probe-holder further comprising a space downstream of the inlet and upstream of the side hollow seat, the probe-holder being characterised in that the space is connected to the side hollow seat through a plurality of two or more inner tubular ducts inclined by an inclination angle α with respect to a transversal plane crossing the first and second side walls of the probe-holder.

According to another aspect of the invention, the inclination angle α of each inner tubular duct with respect to said transversal plane may range from 20° to 30°.

According to a further aspect of the invention, the inclination angle α of each inner tubular duct with respect to said transversal plane may be equal to 25°.

According to an additional aspect of the invention, the side hollow seat may be connected to the longitudinal duct through one or more planar ducts oriented parallel to a longitudinal axis of the longitudinal duct.

According to another aspect of the invention, said one or more planar ducts may be oriented orthogonally to said transversal plane crossing the right and left walls of the probe-holder.

According to a further aspect of the invention, the space may comprise a, optionally threaded, mouth communicating externally.

According to an additional aspect of the invention, the mouth may be connected to the inlet through a transversal duct.

According to another aspect of the invention, the probe-holder may further comprise a transversal duct connected between the inlet of the probe-holder and the longitudinal duct, whereby the transversal duct is configured to operate as by-pass for air mixed with said incoming fluid entering the probe-holder.

According to a further aspect of the invention, the longitudinal duct may be vertical, the side hollow seat may be arranged inferiorly to the longitudinal duct and the outlet of the probe-holder may be superiorly arranged on a side wall selected between the first and the second side walls, whereby the longitudinal duct is configured to receive said flow of a fluid from the bottom upwards.

The advantages offered by the modular probe-holder according to the invention are evident.

In particular, the probe-holder configured to house an amperometric sensor according to the invention allows the solute to be agitated in an efficient way in correspondence to the amperometric sensor for obtaining a reliable and precise concentration measurement.

Moreover, some embodiments of the probe-holder for amperometric sensor according to the invention are provided with a by-pass for air mixed with the fluid entering the probe-holder, that allows to make a degassing of the entering fluid that is particularly important for avoiding malfunctions of the amperometric sensor; in particular, the amount of air mixed with the fluid is larger when the fluid starts to flow in the probe-holder.

The present invention will be now described, by way of illustration and not by way of limitation, according to its preferred embodiments, by particularly referring to the Figures of the annexed drawings, in which:
Figure 1 shows a right side view of a first type of probe-holding module (Fig. 1a), a front view of a second type of probe-holding module (Fig. 1b), a front view of a third type of probe-holding module (Fig. 1c), a front view of a fourth type of probe-holding module (Fig. 1d), and a front view of a fifth type of probe-holding module (Fig. 1e) of a modular probe-holder to which a first embodiment of the probe-holder for amperometric sensor according to the invention is applied;
Figure 2 shows a front perspective view of the first type of probe-holding module (Fig. 2a) and a rear perspective view of the fifth type of probe-holding module (Fig. 2b) of Figure 1;
Figure 3 shows a front view (Fig. 3a) and a cross-section view along the plane AA of Figure 3a (Fig. 3b) of a first assembly of four of the probe-holding modules of Figure 1;
Figure 4 shows a first cross-section view (Fig. 4a) and a second cross-section view (Fig. 4b) of a particular of a coupling zone between two probe-holding modules of Figure 1, wherein each view represents specific features of the coupling;
Figure 5 shows a left side view of the fifth type of probe-holding module (Fig. 5a) of Figure 1 and a cross-section view (Fig. 5b) of a particular of a coupling zone between two probe-holding modules of Figure 1;
Figure 6 shows a front view (Fig. 6a) and a cross-section view along the plane BB of Figure 6a (Fig. 6b) of an assembly of four probe-holding modules of another modular probe-holder to which the first embodiment of the probe-holder for amperometric sensor according to the invention is applied;
Figure 7 shows a cross-section view (Fig. 7a) of the first assembly of Figure 3 wherein each probe-holding module houses a respective sensing probe, an enlargement of the cross-section view (Fig. 7b) and a perspective view of a component coupled to the first assembly (Fig. 7c);
Figure 8 shows a rear perspective view of a second assembly of two of the probe-holding modules of Figure 1 wherein each probe-holding module houses a respective sensing probe;
Figure 9 shows a cross-section view of the first type of probe-holding module of Figure 1; and
Figure 10 shows an enlargement of a portion of the cross-section of the first assembly of Figure 7 (Fig. 10a), a cross-section view along the plane CC of Figure 10a (Fig. 10b) and two enlargements of the same particular of Figure 10b (Fig. 10c and Fig. 10d), wherein each enlargement represents specific features of the particular.

In the Figures identical reference numerals will be used for alike elements.

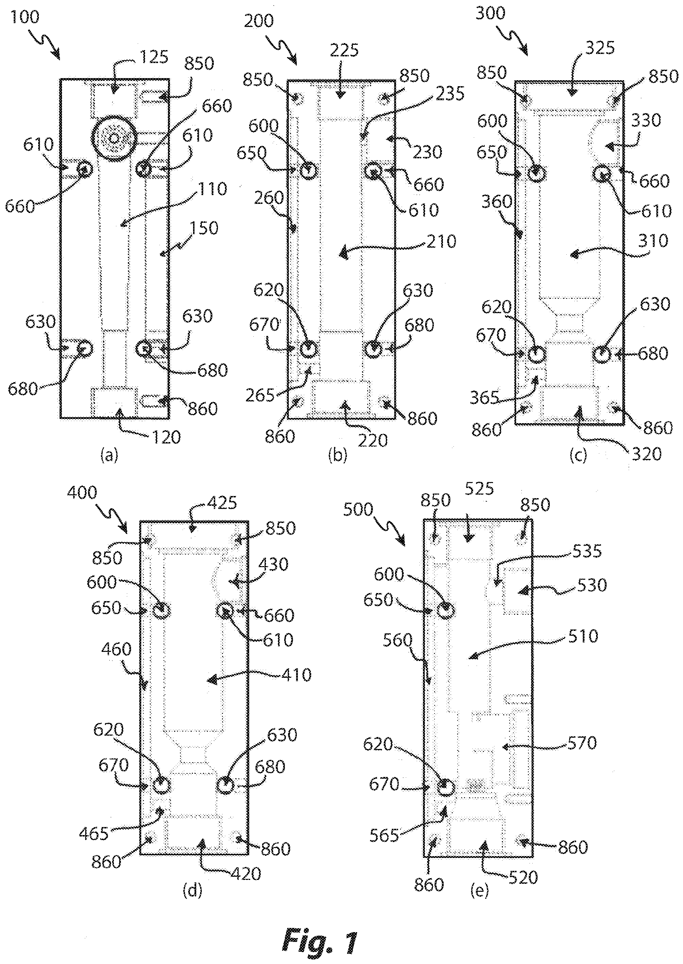

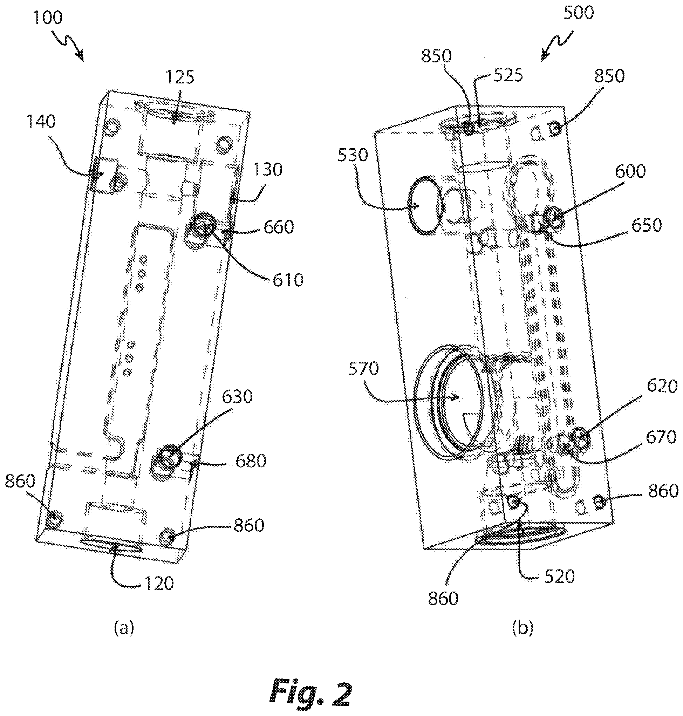

With reference to Figures 1 and 2, it may be observed that a first embodiment of the probe-holder for amperometric sensor according to the invention is a probe-holding module for amperometric sensor applied to a modular probe-holder that may be composed of two or more probe-holding modules coupled to each other the type of which is selected from a group comprising five types of different probe-holding modules, each one made in one piece of plexiglass that can be inscribed in a substantially parallelepiped-shaped polyhedron, and capable to be coupled to each other.
A first type of probe-holding module 100, shown in Figures 1a and 2a, is configured to house an adjustable flow meter, optionally capable to measure a volumetric flow rate ranging from 10 to 100 litres/hour. The first probe-holding module 100 comprises a longitudinal central duct (i.e., a vertical central duct) 110 that communicates externally through a lower threaded mouth 120, through an upper hollow threaded seat 125 and through an upper right side threaded outlet 130 ("right side" with respect to the front view of the first probe-holding module 100) connected to the longitudinal central duct 110 through an upper right transverse duct 135. Moreover, the first probe-holding module 100 is provided with an upper left side hollow seat 140 and a rear hollow seat 150.
A second type of probe-holding module 200, shown in Figure 1b, is configured to house a sensing probe and comprises a longitudinal central duct 210 having a first diameter, optionally equal to 12 mm, that communicates externally through a lower threaded mouth 220, through an upper hollow threaded seat 225 and through an upper right side threaded outlet 230 ("right side" with respect to the front view of the second probe-holding module 200) connected to the longitudinal central duct 210 through an upper right transverse duct 235. Moreover, the second probe-holding module 200 is provided with a groove 260 on the left side wall that communicates with the longitudinal central duct 210 through a lower left transverse duct 265.
A third type of probe-holding module 300, shown in Figure 1c, is configured to house a sensing probe and comprises a longitudinal central duct 310 having a second diameter larger than the first diameter, optionally equal to 24 mm, that communicates externally through a lower threaded mouth 320, through an upper hollow threaded seat 325 and through an upper right side threaded outlet 330 ("right side" with respect to the front view of the third probe-holding module 300). Moreover, the third probe-holding module 300 is provided with a groove 360 on the left side wall that communicates with the longitudinal central duct 310 through a lower left transverse duct 365.
A fourth type of probe-holding module 400, shown in Figure 1d, is configured to house a sensing probe and comprises a longitudinal central duct 410 having a third diameter larger than the second diameter, optionally equal to 35 mm, that communicates externally through a lower threaded mouth 420, through an upper hollow threaded seat 425 and through an upper right side threaded outlet 430 ("right side" with respect to the front view of the fourth probe-holding module 400). Moreover, the fourth probe-holding module 400 is provided with a groove 460 on the left side wall that communicates with the longitudinal central duct 410 through a lower left transverse duct 465.

The probe-holding module for amperometric sensor according to the invention is a fifth type of probe-holding module 500, shown in Figures 1e and 2b, that is configured to house a horizontal amperometric sensor in a lower right side hollow seat 570 communicating inferiorly with a lower threaded mouth 520, in turn communicating externally, and superiorly with a longitudinal duct 510, in turn communicating externally through an upper hollow threaded seat 525 and through an upper right side threaded outlet 530 ("right side" with respect to the front view of the fifth probe-holding module 500). Moreover, the fifth probe-holding module 500 is provided with a groove 560 on the left side wall that communicates with the lower right side hollow seat 570 through a lower left transverse duct 565.

The fastening of the probe-holding modules is guaranteed by ties constrained in the probe-holding modules operated by grub screws, allowing the coupling between probe-holding modules without constraints of consecutiveness apart from the first type of probe-holding module 100 that, in the modular probe-holders shown in the Figures to which the first embodiment of the probe-holder for amperometric sensor according to the invention, must be always the first module of the series of assembled modules which form the modular probe-holder and apart from the fifth type of probe-holding module 500 that, in the modular probe-holders shown in the Figures to which the first embodiment of the probe-holder for amperometric sensor according to the invention is applied, must be always the last module of the series of assembled modules which form the modular probe-holder.

To this end, the second, the third and the fourth type of probe-holding modules 200, 300 and 400 are provided on the front and rear walls with a pair of upper left threaded holes 600 communicating with a pair of respective upper left side seats 650 communicating externally and having longitudinal axis orthogonal to the axis of the respective threaded hole 600, a pair of upper right threaded holes 610 communicating with a pair of respective upper right side seats 660 communicating externally and having longitudinal axis orthogonal to the axis of the respective threaded hole 610, a pair of lower left threaded holes 620 communicating with a pair of respective lower left side seats 670 communicating externally and having longitudinal axis orthogonal to the axis of the respective threaded hole 620, and a pair of lower right threaded holes 630 communicating with a pair of respective lower right side seats 680 communicating externally and having longitudinal axis orthogonal to the axis of the respective threaded hole 630; each one of the threaded holes is configured to receive a grub screw, while each one of the seats communicating with the threaded holes is configured to partially receive a tie.

The first type of probe-holding module 100 is provided on the front and rear walls with only the pair of upper right threaded holes 610 and the pair of lower right threaded holes 630, each one of which is also configured to receive a grub screw, communicating with respective upper and lower right side seats 660 and 680, each one of which is also configured to partially receive a tie.

The fifth type of probe-holding module 500 is provided on walls front and rear walls with only the pair of upper left threaded holes 600 and the pair of lower left threaded holes 620, each one of which is also configured to receive a grub screw, communicating with respective upper and lower left side seats 650 and 670, each one of which is also configured to partially receive a tie.

However, it should be understood that other embodiments of the probe-holder for amperometric sensor according to the invention may consist of a probe-holding module for amperometric sensor, for instance a horizontal amperometric sensor housed in a front hollow seat or a vertical amperometric sensor housed in a corresponding seat, that may be coupled to other modules in any position along the series of assembled modules forming the modular probe-holder (i.e. not necessarily placed as last module); in this case, such probe-holding modules configured to house amperometric sensors are provided with the same assembly of pairs of threaded holes 600, 610, 620 and 630 and of communicating seats 650, 660, 670 and 680 with which the second, the third and the fourth type of probe-holding modules 200, 300 and 400 are provided.

Moreover, other modular probe-holders may have probe-holding modules configured to house flow meters which may be coupled to other modules in any position along the series of assembled modules forming the modular probe-holder (i.e. not necessarily placed as first module); such probe-holding modules configured to house flow meters are also provided with the same assembly of pairs of threaded holes 600, 610, 620 and 630 and of communicating seats 650, 660, 670 and 680 with which the second, the third and the fourth type of probe-holding modules 200, 300 and 400 are provided.

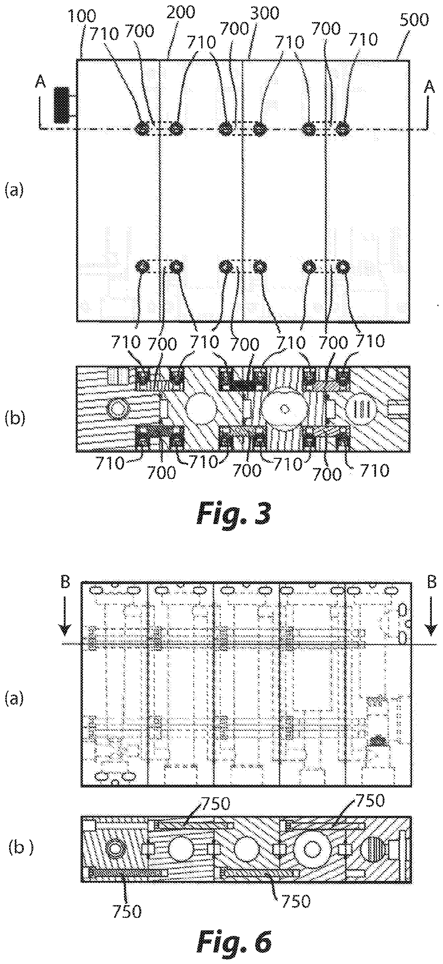

With reference to Figure 3, wherein by way of example and not by way of limitation a modular probe-holder constituted of an ordered series of a first type, a second type, a third type and a fifth type of probe-holding modules 100, 200, 300 and 500 is shown, it may be observed that the ties 700 are inserted into the pairs of facing side seats 650, 660, 670 and 680 of two adjacent probe-holding modules and afterwards the grub screws 710 are screwed into the threaded holes 600, 610, 620 and 630, causing the adjacent probe-holding modules to get closer. As stated, such fastening is present on both front and rear faces of the probe-holding modules.

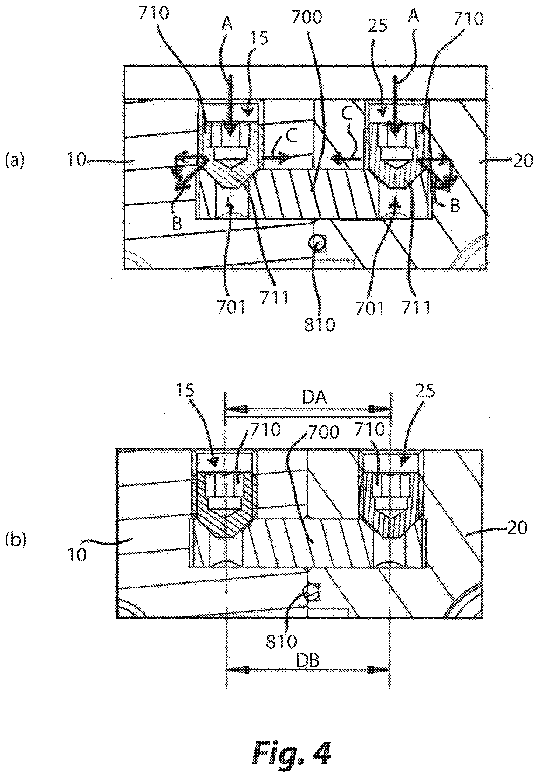

In order to better understand the operation of the grub screws 700 and ties 710 reference can be made to Figure 4, wherein an enlargement of a cross-section of a coupling zone of two adjacent probe-holding modules (generically indicated with the reference numerals 10 and 20) comprising a tie 700 inserted into a pair of corresponding facing side seats is shown. The tie 700 comprises two holes 701 each configured to receive the tip 711 of a grub screw 710 screwed into a respective threaded hole (the threaded holes are generically indicated with the reference numerals 15 and 25). In particular, the tip 711 of a grub screw has a conical lateral surface, optionally with apex angle equal to 90° (whereby the side surface has an inclination of 45° with respect to the longitudinal axis of the grub screw 710), and the hole 701 and tie 700 have a corresponding support inclined surface, optionally with inclination of 45° with respect to the longitudinal axis of the hole 701.

The area of contact between tip 711 of the grub screw 710 and hole 701 of the tie 700 only comprises the portion of the support inclined surface of the hole 701 of the tie 700 that is farthest from the adjacent probe-holding module (20 or 10). To this end, when the tie 700 is symmetrically arranged in the pair of corresponding facing side seats, there is an offset between the longitudinal axis of each one of the threaded holes 15 and 20 (coinciding with the longitudinal axis of the grub screw 710 inserted into such threaded hole) and the longitudinal axis of the hole 701 of the tie 700; optionally, when the two adjacent probe-holding modules 10 and 20 are coupled, the distance DA between the longitudinal axes of the two threaded holes 15 and 20 (communicating with the pair of side seats facing the two adjacent probe-holding modules 10 and 20) is larger than the distance DB between the longitudinal axes of the holes 701 of the tie 700, optionally by an amount ranging from 2% to 3%, more optionally by an amount equal to 2,5% (whereby DA=1,025*DB).

When a grub screw 710 is screwed into the respective threaded hole (15 or 25), it advances longitudinally (along the direction of the arrow A) exerting a force (along the direction of the arrow B) perpendicular to the support surface of the respective hole 701 of the tie 700. The preferred inclination angle of the side surface of the tip 711 of the grub screw, equal to 45°, maximises the area of contact between tip 711 of the grub screw 710 and hole 701 of the tie 700 dedicated to the transmission of forces. The horizontal component of such force produces a constraint reaction (along the direction of the arrow C) on the portion of the surface of the threaded hole (15 or 25) closest to the adjacent probe-holding module (20 or 10), causing the two adjacent probe-holding modules 10 and 20 to get closer to each other.

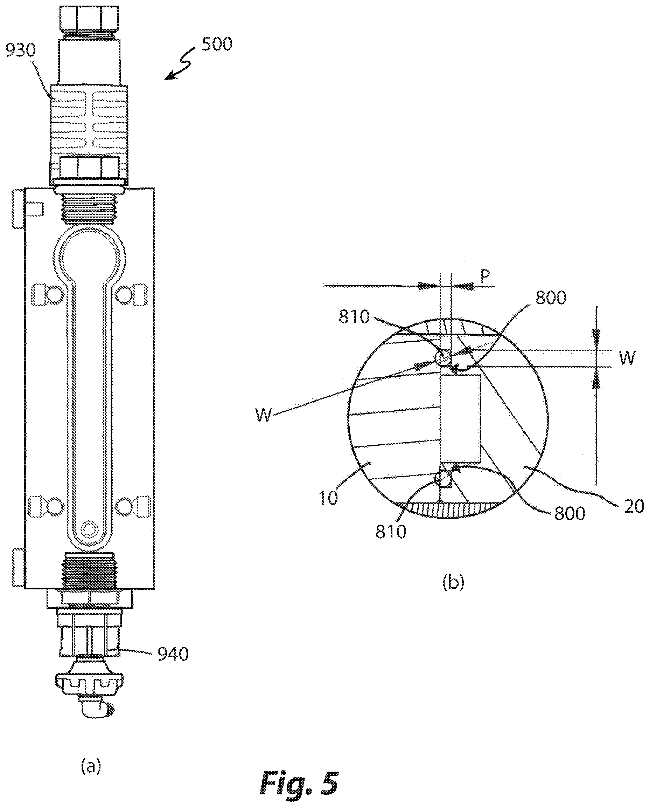

With reference to Figures 2b and 5a, wherein by way of example and not by way of limitation a probe-holding module 500 of the fifth type configured to house an amperometric sensor (that constitutes the first embodiment of the probe-holder for amperometric sensor according to the invention) is shown, it may be observed that on the left side wall of the probe-holding modules is present a seal groove 800 arranged so as to surround the groove 560 of the probe-holding module 500 and, when the latter is coupled to an adjacent probe-holding module (100, 200, 300 or 400), also the upper left side outlet (130, 230, 330 or 430) on the left side wall of the adjacent probe-holding module (100, 200, 300 or 400). The seal groove 800 is configured to house an O-ring seal 810 of elastic material, optionally of fluoroelastomer known as FPM or FKM, with optionally circular cross-section, whereby the seal 810 is capable to ensure the sealing of the coupling between adjacent probe-holding modules since it surrounds the path of the fluid when passing from one to the other of the two adjacent probe-holding modules.

As shown in Figure 5b, wherein an enlargement of a cross-section of a coupling zone of two adjacent probe-holding modules (generically indicated with the reference numerals 10 and 20) comprising a groove 800 housing a seal 810 is shown, the geometry and size of the groove 800 and seal 810 are such that they guarantee the compression of the seal 810 when the two adjacent probe-holding modules 10 and 20 are coupled, so as to compensate planarity errors (i.e. irregularities of the opposing surfaces) of the facing walls of the two adjacent probe-holding modules 10 and 20. In this way, the seal 810 ensures the correct operation of the modular probe-holder with both fluid in static conditions and moving flow under several operation conditions, for instance at a first condition with fluid temperature of 25°C and fluid pressure of 10 bar and at a second condition with fluid temperature of 70°C and fluid pressure of 7 bar. Advantageously, the deformation of the seal 810 mainly concerns the direction orthogonal to the walls of contact of two adjacent probe-holding modules 10 and 20. Optionally, the groove 800 has rectangular cross-section, with depth p and width w equal to the diameter w of the seal 810 with circular cross-section, wherein p ranges from 65% to 75% of w (whereby 0.65*w ≤ p ≤ 0,7*w), p being more optionally equal to 70% of w (whereby p=0,7*w). In this case, even when the single probe-holding modules provided with the seal housed in the groove are disassembled, the seal stably remains in the groove even if the module is moved. In other words, the sealing effect is given by the deformation of the seal 810 during fastening of two adjacent probe-holding modules 10 and 20. The specific torque of fastening of the grub screws 710 and the absence of vibrations during the operation of the modular probe-holder do not render the use of self-locking devices necessary and ensure the compression imposed to the seal 810 for the entire lifetime of the modular probe-holder. Optionally, the hardness of the seal is not lower than 50 shore A (SHA), more optionally not lower than 60 SHA, in order to avoid that the seal is extruded between the walls of the two adjacent probe-holding modules 10 and 20.

In the modular probe-holders shown in the Figures to which the first embodiment of the probe-holder for amperometric sensor according to the invention is applied, since the first type of probe-holding module 100 is the first module of the series of assembled modules forming the modular probe-holder, such first type of probe-holding module 100 is not provided on the left side wall with the seal groove 800.

Other modular probe-holders may have the probe-holding modules having a seal groove on the right side wall, instead of the left side wall, still arranged so as to surround the path of the fluid when passing from one to the other of the two adjacent probe-holding modules. In the case where the fifth type of probe-holding module 500 is still always the last module of the series of assembled modules forming a modular probe-holder, such fifth type of probe-holding module 500 would not be provided on the right side wall with such seal groove.

Further modular probe-holders may have the probe-holding modules which have a seal groove on both the left side wall and the right side wall, configured such that they do not overlap with the seal groove respectively on the right side wall and the left side wall of two other adjacent modules, both the seal grooves on the two side walls being arranged so as to surround the path of the fluid when passing from one to the other of the two adjacent probe-holding modules.

It should be understood that the specific arrangement of the seal groove configured to house an O-ring seal, illustrated with reference to Figures 2b and 5, is not a feature essential to the probe-holder for amperometric sensor according to the invention, that may be made as monolithic probe-holder configured to house only an amperometric sensor or to house an amperometric sensor and one or more further sensing probes, optionally in respective housings communicating with each other.

It should be further understood that the mechanical means for coupling two probe-holding modules is not a feature essential to the probe-holder for amperometric sensor according to the invention, and it can be different from that illustrated with reference to the Figures also in function of the type, shape and size of the modules which have to be coupled. By way of example, and not by way of limitation, such coupling mechanical means may also comprise or consist of at least one flange and/or rivets and/or screws and bolts.

By way of example, reference to Figure 6, it may be observed that the coupling mechanical means used in another modular probe-holder, to which the first embodiment of the probe-holder for amperometric sensor according to the invention, comprises, instead of grub screws and ties, threaded studs 750 screwed in suitable transverse through holes (operating as side seats) accessible on the side walls of the single probe-holding modules. Differently from the solution adopted in the modular probe-holder shown in Figure 3 and 4, the coupling through threaded studs 750 imposes that, in order to access a probe-holding module of a series of assembled modules forming the modular probe-holder, it is necessary to disassemble all the subsequent probe-holding modules.

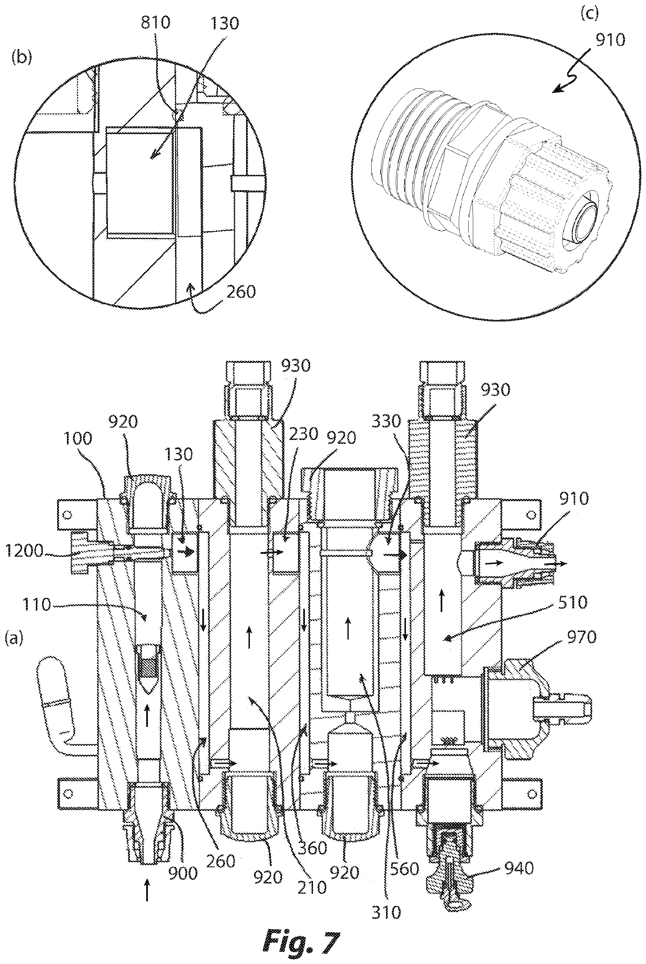

Figure 7, wherein by way of example and not by way of limitation a modular probe-holder constituted by an ordered series of a first type, a second type, a third type and a fifth type of probe-holding module 100, 200, 300, and 500 (illustrated with reference to Figures 1 and 2) is shown, each one of which houses a respective sensing probe, shows the path of the water flow (schematically represented by the arrows in Figure 7) in the modular probe-holder to which the first embodiment of the probe-holder for amperometric sensor according to the invention is applied. In particular, the entrance of the water flow in the modular probe-holder occurs through a tube-holder 900 coupled in the lower threaded mouth 120 of the first probe-holding module 100. The longitudinal ducts 110, 210, 310 and 510 of the probe-holding modules 100, 200, 300, and 500 guarantee that the fluid is moved from the bottom upwards inside each probe-holding module and that it has a constant velocity along the entire probe-holding module. The exit of the flow from each probe-holding module always occurs from the right wall of the same module, in particular from the upper right side outlets 130, 230, 330 and 530 of the probe-holding modules 100, 200, 300, and 500. The path of the fluid when passing from the previous to the next of two adjacent probe-holding modules occurs along the grooves on the left side walls 260, 360 and 560 of the next probe-holding module, as shown by the enlarged particular of Figure 7b related to the passage from the second probe-holding module 200 to the third probe-holding module 300. The flow exiting from the last probe-holding module of the series, that in Figure 7 is the fifth probe-holding module 500, is channelled by screwing a tube-holder 910 (shown enlarged in Figure 7c) into the upper right side outlet. The lower threaded mouths 220, 320 and 520 of the second, third and fifth probe-holding modules 200, 300, and 500, as well as the upper threaded mouths 125, 225, 325 and 525 of all the probe-holding modules 100, 200, 300, and 500 are closed by caps 920 and other devices 930 and 940 (e.g. electrodes and taps), whereby they do not provide the flow shown in Figure 7 with other exits.

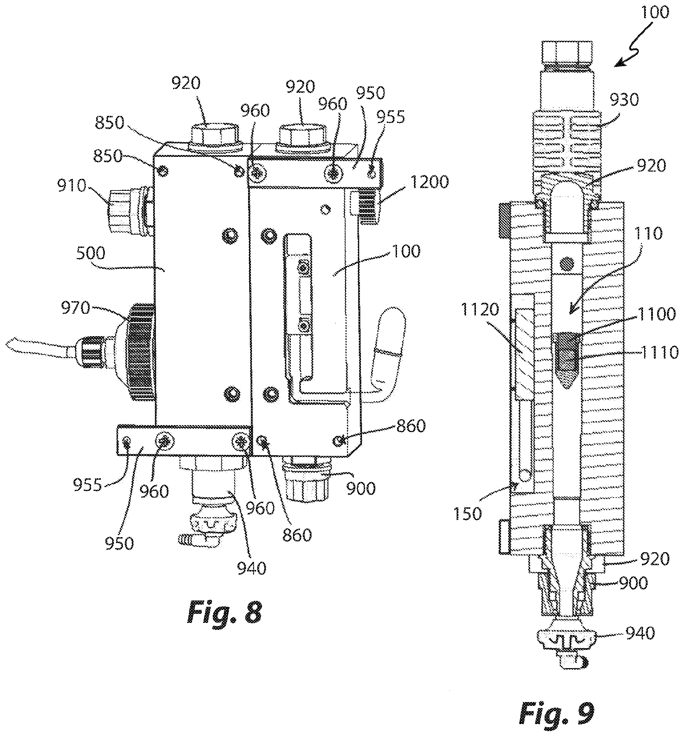

Making reference again to Figures 1 and 2, all the probe-holding modules are provided, on the rear wall, with a pair of upper rear threaded holes 850 and with a pair of lower rear threaded holes 860 configured to receive a screw for fastening rear brackets allowing the modular probe-holder to be fixed to a wall or support plate. Such rear brackets are fixed to the probe-holding modules at the ends of the series of assembled modules forming the modular probe-holder. By way of example, and not by way of limitation, Figure 8 shows a modular probe-holder constituted by a first type and a fifth type of probe-holding modules 100 and 500 to which two rear brackets 950 have been fixed through screws 960 inserted into the rear threaded holes 850 and 860; the rear brackets 950 are provided with holes 955 for the fastening through conventional means, such as Fischer plugs, to a wall or support plate.

The probe-holding modules allow to make modular probe-holders having any configuration.

The probe-holding module 100 of the first type is configured to house a flow meter dedicated to the measure of volumetric flow rate, optionally indicated by a suitably calibrated graduated scale present on the front wall of the probe-holding module 100. Making reference to Figure 9, it may be observed that a float 1100, optionally of plastic material, is advantageously inserted into the longitudinal central duct 110, that is provided with a permanent magnet 1110 configured to interact with a Reed switch 1120 housed in the rear hollow seat 150 of the probe-holding module 100; in particular, the Reed switch 1120 is actuated by the permanent magnet 1110 (that is inserted into the float 1100) when the volumetric flow rate has a value in a predetermined range, optionally ranging from 60 l/h to 80 l/h.

Other modular probe-holder according to the invention may comprise, instead of the Reed switch 1120, a different proximity sensor interacting with the float 1100, such as for instance a Hall effect sensor, that senses when the volumetric flow rate at the inlet of the modular probe-holder is not within a predetermined range even different from that ranging from 60 l/h to 80 l/h, optionally a predetermined range ranging from 60 l/h to 100 l/h.

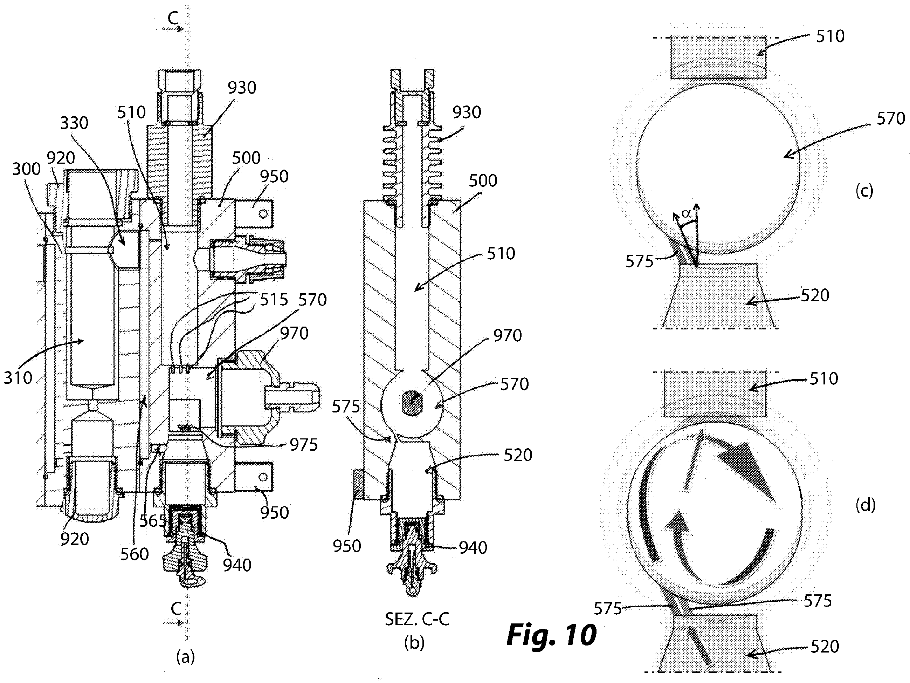

As shown in Figures 10a and 10b, the probe-holding module 500 of the fifth type, that constitutes the first embodiment of the probe-holder for amperometric sensor according to the invention, is configured to house a horizontal amperometric sensor 970 in the lower right side hollow seat 570, having a circular or ellipsoidal transverse section (as shown in Figure 10b), that inferiorly communicates with the lower threaded mouth 520 through a plurality of inner tubular ducts 575 (in the preferred embodiment of Figure 10, such inner tubular ducts 575 are seven, arranged along two rows visible in Figures 10c and 10d as aligned sideways, of which the outlet mouths 975 are visible in Figure 10a in the lower right side hollow seat 570), the diameter of which (optionally equal to 2 mm) is significantly lower than its length (optionally equal to 12 mm).

The probe-holding module 500, that constitutes the first embodiment of the probe-holder for amperometric sensor according to the invention, is provided with an upper left transverse duct 580 (that is optionally tubular) that, in correspondence with the upper right side threaded outlet 330 of the adjacent module 300, puts in communication the upper portion of the groove 560 on the left side wall with the upper portion of the longitudinal duct 510. Such upper left transverse duct 580 allows the air present in the fluid entering the groove 560 on the left side wall to be eliminated by the Venturi effect; in other words, the upper left transverse duct 580 operates as by-pass for air mixed with the fluid entering the fifth probe-holding module 500. In this way, the upper left transverse duct 580 achieves a degassing of the fluid entering the fifth probe-holding module 500 that is particularly important for avoiding malfunctions of the amperometric sensor 970; in particular, the amount of air mixed with the fluid is larger when the fluid starts to flow in the modular probe-holder.

As shown in greater detail in Figures 10c and 10d, the water flow (schematically represented in Figure 10d by the arrows) in the probe-holding module 500 (that constitutes the first embodiment of the probe-holder for amperometric sensor according to the invention) is channelled from the bottom upwards in the lower right side hollow seat 570 through the inner tubular ducts 575 each having an inclination angle α, with respect to the transverse plane crossing the right and left side walls of the probe-holding module 500, optionally ranging from 20° to 30°, more optionally equal to 25°. This channelling, by accelerating the flow, allows the solute to be agitated and further guarantees the entrainment of the balls (optionally of plastics, glass or ceramic, not shown) housed in the lower right side hollow seat 570 for cleaning the electrode of the amperometric sensor. Moreover, the lower right side hollow seat 570 communicates superiorly with the longitudinal duct 510, through a plurality (Figure 10 shows three) of planar ducts 515 (i.e. having a dimension much lower than the other two), oriented parallel to the longitudinal axis of the longitudinal duct 510 (and optionally orthogonally to the transverse plane crossing the right and left side walls of the probe-holding module 500).

In particular, the orientation of the longitudinal duct 510 in vertical direction is not a feature essential to the probe-holder for amperometric sensor according to the invention.

The upper left transverse duct 580 is not a feature essential to the probe-holder for amperometric sensor according to the invention. In particular, the probe-holder for amperometric sensor according to the invention may comprise any transverse duct, connected between an inlet of the probe-holder and the longitudinal duct, that operates as by-pass for air mixed with the fluid entering the probe-holder.

Similarly, the planar ducts 515 are not a feature essential to the probe-holder for amperometric sensor according to the invention, and they may be replaced with any other structure of ducts.

Also, the specific arrangement of the groove 560, the lower left transverse duct 565 and the lower threaded mouth 520, communicating with each other, is not a feature essential to the probe-holder for amperometric sensor according to the invention. In particular, such specific arrangement may be generalised as any space (that may also comprise or consist of one or more ducts shaped anyway) connected downstream of an inlet of the probe-holder for amperometric sensor according to the invention and upstream of a side hollow seat (that may be located on the right or left or even frontally), that is configured to house an amperometric sensor, that communicates with such side hollow seat through internal tubular ducts similar to the ducts 575 connecting the lower threaded mouth 520 to the lower side hollow seat 570 shown in Figure 10.

Furthermore, the groove 560 is not a feature essential to the probe-holder for amperometric sensor according to the invention, and it may be replaced with any type of inlet.

Again, it should be understood that the specific arrangement of the internal tubular ducts 575, illustrated with reference to Figure 10, is also applicable (even independently from the arrangement of the upper left transverse duct 580) to other embodiments of the probe-holder for amperometric sensor according to the invention different from the first embodiment shown in the Figures. By way of example, and not by way of limitation, such arrangement of the internal tubular ducts 575 may be applied in a monolithic probe-holder for amperometric sensor, that is configured to house only an amperometric sensor or to house an amperometric sensor and one or more further sensing probes, optionally in respective housings communicating with each other.

The preferred embodiments of this invention have been described and a number of variations have been suggested hereinbefore, but it should be understood that those skilled in the art can make other variations and changes, without so departing from the scope of protection thereof, as defined by the attached claims.

## Claims

1. Probe-holder (500), having a first and a second side walls, configured to house an amperometric sensor (970) in a side hollow seat (570), the probe-holder comprising an inlet (560) and an outlet (530) and a longitudinal duct (510) connected downstream of the side hollow seat (570), the probe-holder (500) being configured to receive a flow of a fluid from the inlet (560) to the outlet (530), the probe-holder (500) further comprising a space (520) downstream of the inlet (560) and upstream of the side hollow seat (570), the probe-holder (500) being **characterised in that** the space (520) is connected to the side hollow seat (570) through a plurality of two or more inner tubular ducts (575) inclined by an inclination angle α with respect to a transversal plane crossing the first and second side walls of the probe-holder (500).

2. Probe-holder (500) according to claim 1, **characterised in that** the inclination angle α of each inner tubular duct (575) with respect to said transversal plane ranges from 20° to 30°.

3. Probe-holder (500) according to claim 2, **characterised in that** the inclination angle α of each inner tubular duct (575) with respect to said transversal plane is equal to 25°.

4. Probe-holder (500) according to any one of the preceding claims, **characterised in that** the side hollow seat (570) is connected to the longitudinal duct (510) through one or more planar ducts (515) oriented parallel to a longitudinal axis of the longitudinal duct (510).

5. Probe-holder (500) according to claim 4, **characterised in that** said one or more planar ducts (515) are oriented orthogonally to said transversal plane crossing the right and left walls of the probe-holder (500).

6. Probe-holder (500) according to any one of the preceding claims, **characterised in that** the space comprises a, optionally threaded, mouth (520) communicating externally.

7. Probe-holder (500) according to claim 6, **characterised in that** the mouth (520) is connected to the inlet (560) through a transversal duct (565).

8. Probe-holder (500) according to any one of the preceding claims, **characterised in that** it further comprises a transversal duct (580) connected between the inlet of the probe-holder and the longitudinal duct (510), whereby the transversal duct (580) is configured to operate as by-pass for air mixed with said incoming fluid entering the probe-holder (500).

9. Probe-holder (500) according to any one of the preceding claims, **characterised in that** the longitudinal duct (510) is vertical, the side hollow seat (570) is arranged inferiorly to the longitudinal duct (510) and the outlet (530) of the probe-holder (500) is superiorly arranged on a side wall selected between the first and the second side walls, whereby the longitudinal duct (510) is configured to receive said flow of a fluid from the bottom upwards.

## Patentansprüche

1. Sondenhalter (500), mit einer ersten und einer zweiten Seitenwand, die dazu konfiguriert ist, einen amperometrischen Sensor (970) in einem seitlichen hohlen Sitz (570) unterzubringen, wobei der Sondenhalter einen Einlass (560) und einen Auslass (530) und einen longitudinalen Kanal (510) umfasst, der stromabwärts des seitlichen hohlen Sitzes (570) angeschlossen ist, wobei der Sondenhalter (500) dazu konfiguriert ist, den Fluss eines Fluids von dem Einlass (560) zu dem Auslass (530) zu empfangen, wobei der Sondenhalter (500) ferner einen Raum (520) stromabwärts des Einlasses (560) und stromaufwärts des seitlichen hohlen Sitzes (570) umfasst, wobei der Sondenhalter (500) **dadurch gekennzeichnet ist, dass** der Raum (520) durch eine Mehrzahl von zwei oder mehr inneren, röhrenförmigen Kanälen (575), die um einen Neigungswinkel α in Bezug auf eine transversale Ebene geneigt sind, die die erste und die zweite Seitenwand des Sondenhalters (500) kreuzt, mit dem seitlichen hohlen Sitz (570) verbunden ist.

2. Sondenhalter (500) nach Anspruch 1, **dadurch gekennzeichnet, dass** der Neigungswinkel α eines jeden inneren, röhrenförmigen Kanals (575) in Bezug auf die genannte transversale Ebene in einem Bereich von 20° bis 30° liegt.

3. Sondenhalter (500) nach Anspruch 2, **dadurch gekennzeichnet, dass** der Neigungswinkel α einer jeden röhrenförmigen Leitung (575) in Bezug auf die genannte transversale Ebene gleich 25° ist.

4. Sondenhalter (500) nach einem der vorhergehenden Ansprüche, **dadurch gekennzeichnet, dass** der seitliche hohle Sitz (570) mit dem longitudinalen Kanal (510) durch einen oder mehrere planare Kanäle (515) verbunden ist, die parallel zu einer Längsachse des longitudinalen Kanals (510) orientiert ist.

5. Sondenhalter (500) nach Anspruch 4, **dadurch gekennzeichnet, dass** der eine oder die mehreren planaren Kanäle (515) orthogonal zur genannten transversalen Ebene, die die rechte und die linke Wand des Sondenhalters (500) kreuzt, orientiert ist.

6. Sondenhalter (500) nach einem der vorhergehenden Ansprüche, **dadurch gekennzeichnet, dass** der Raum einen, optional mit einem Gewinde versehenen, Mund (520) umfasst, der nach außen kommuniziert.

7. Sondenhalter (500) nach Anspruch 6, **dadurch gekennzeichnet, dass** der Mund (520) mit dem Einlass (560) durch einen transversalen Kanal (565) verbunden ist.

8. Sondenhalter (500) nach einem der vorhergehenden Ansprüche, **dadurch gekennzeichnet, dass** er ferner einen transversalen Kanal (580) umfasst, der zwischen dem Einlass des Sondenhalters und dem longitudinalen Kanal (510) verbunden ist, wobei der transversale Kanal (580) konfiguriert ist, als Umleitung für Luft zu wirken, die mit dem genannten einlaufenden Fluid, welches in den Sondenhalter (500) eintritt, gemischt ist.

9. Sondenhalter (500) nach einem der vorhergehenden Ansprüche, **dadurch gekennzeichnet, dass** der longitudinale Kanal (510) vertikal ist, der seitliche hohle Sitz (570) unterhalb des longitudinalen Kanals (510) angeordnet ist, und der Auslass (530) des Sondenhalters (500) oberhalb an der ersten oder der zweiten Seitenwand angeordnet ist, wobei der longitudinale Kanal (510) konfiguriert ist, den genannten Fluss eines Fluids vom Boden nach oben zu empfangen.

## Revendications

1. Support de tube à échantillons (500), ayant des première et seconde parois latérales, configuré pour loger un capteur ampèremétrique (970) dans un logement creux latéral (570), le support de tube à échantillons comprenant un orifice d'entrée (560) et un orifice de sortie (530) et un conduit longitudinal (510) connecté en aval du logement creux latéral (570), le support de tube à échantillons (500) étant configuré pour recevoir un écoulement d'un fluide de l'orifice d'entrée (560) à l'orifice de sortie (530), le support de tube à échantillons (500) comprenant en outre un espace (520) en aval de l'orifice d'entrée (560) et en amont du logement creux latéral (570), le support de tube à échantillons (500) étant **caractérisé en ce que** l'espace (520) est connecté au logement creux latéral (570) par le biais d'une pluralité de deux conduits tubulaires internes ou plus (575) inclinés selon un angle d'inclinaison α par rapport à un plan transversal traversant les première et seconde parois latérales du support de tube à échantillons (500).

2. Support de tube à échantillons (500) selon la revendication 1, **caractérisé en ce que** l'angle d'inclinaison α de chaque conduit tubulaire interne (575) par rapport audit plan transversal est situé dans la plage de 20° à 30°.

3. Support de tube à échantillons (500) selon la revendication 2, **caractérisé en ce que** l'angle d'inclinaison α de chaque conduit tubulaire interne (575) par rapport audit plan transversal est égal à 25°.

4. Support de tube à échantillons (500) selon l'une quelconque des revendications précédentes, **caractérisé en ce que** le logement creux latéral (570) est connecté au conduit longitudinal (510) par le biais d'un ou de plusieurs conduits planaires (515) orientés parallèlement à un axe longitudinal du conduit longitudinal (510).

5. Support de tube à échantillons (500) selon la revendication 4, **caractérisé en ce que** ledit un ou plusieurs conduits planaires (515) sont orientés orthogonalement audit plan transversal traversant les parois droite et gauche du support de tube à échantillons (500).

6. Support de tube à échantillons (500) selon l'une quelconque des revendications précédentes, **caractérisé en ce que** l'espace comprend une bouche (520), en variante filetée, communiquant avec l'extérieur.

7. Support de tube à échantillons (500) selon la revendication 6, **caractérisé en ce que** la bouche (520) est connectée à l'orifice d'entrée (560) par le biais d'un conduit transversal (565).

8. Support de tube à échantillons (500) selon l'une quelconque des revendications précédentes, **caractérisé en ce qu'**il comprend un conduit transversal (580) connecté entre l'orifice d'entrée du support de tube à échantillons et le conduit longitudinal (510), moyennant quoi le conduit transversal (580) est configuré pour fonctionner en tant que dérivation pour de l'air mélangé audit fluide arrivant entrant dans le support de tube à échantillons (500).

9. Support de tube à échantillons (500) selon l'une quelconque des revendications précédentes, **caractérisé en ce que** le conduit longitudinal (510) est vertical, le logement creux latéral (570) est agencé à une position inférieure au conduit longitudinal (510) et l'orifice de sortie (530) du support de tube à échantillons (500) est agencé à une position supérieure sur une paroi latérale sélectionnée entre les première et seconde parois latérales, moyennant quoi le conduit longitudinal (510) est configuré pour recevoir ledit écoulement d'un fluide remontant depuis le fond.
